# EUROPEAN PATENT SPECIFICATION

(11) **EP 2 158 921 B1**
(45) Date of publication and mention of the grant of the patent: **04.01.2012**
(21) Application number: 09178084.1
(22) Date of filing: 21.03.2007
(51) Int. Cl.: A61K 39/145

(54) **Intranasal influenza vaccine based on virosomes**
Intranasale Influenzaimpfstoffe aus Virosomen
Vaccine intranasal pour la grippe à partir de virosomes

(30) Priority: 22.03.2006 US 784462 P; 22.03.2006 EP 06111534
(43) Date of publication of application: 03.03.2010
(62) Divisional of application: 07727166.6
(73) Proprietor: Abbott Biologicals B.V., 1381 CP Weesp (NL)
(72) Inventor: Kersten, Alexander J., 1381 CP Weesp (NL); Gerez, Lisya, 1381 CP Weesp (NL); Schoen, Pieter J., 1381 CP Weesp (NL); Nauta, Jozef J.P, 1381 CP Weesp (NL); van Rheineck Leyssius, Dorine, 1381 CP Weesp (NL)
(74) Representative: Prüfer & Partner GbR European Patent Attorneys

(56) References cited:
- WO-A-2004/110486
- GLÜCK U ET AL: "Phase 1 evaluation of intranasal virosomal influenza vaccine with and without Escherichia coli Heat-Labile Toxin in adult volunteers" JOURNAL OF VIROLOGY, THE AMERICAN SOCIETY FOR MICROBIOLOGY, US, vol. 73, no. 9, September 1999 (1999-09), pages 7780-7786, XP002140449 ISSN: 0022-538X
- DURRER P ET AL: "Mucosal antibody response induced with a nasal virosome-based influenza vaccine" VACCINE, BUTTERWORTH SCIENTIFIC. GUILDFORD, GB, vol. 21, no. 27-28, 1 October 2003 (2003-10-01), pages 4328-4334, XP004458029 ISSN: 0264-410X
- DE BRUIJN ET AL: "The virosomal influenza vaccine Invivac<(>R): Immunogenicity and tolerability compared to an adjuvanted influenza vaccine (Fluad<(>R)) in elderly subjects" VACCINE, BUTTERWORTH SCIENTIFIC. GUILDFORD, GB, vol. 24, no. 44-46, 9 November 2006 (2006-11-09), pages 6629-6631, XP005755546 ISSN: 0264-410X

## Description

### Field of the invention

The present invention relates, for example, to compositions for inactivated influenza vaccines and routes of administration wherein a single intranasal or inhalational administration yields a systemic immune response that is positively correlated to clinical protection.

### Background of the invention

Various concepts for immunization against influenza via the nasal or oropharyngeal route and using inactivated influenza antigen have been explored as needle-less alternatives to the subcutaneous or intramuscular immunization. Experimental data supportive for needle-less approaches have been generated in animal models. Concepts using inactivated influenza antigen (such as chemically inactivated whole virus particles, or further processed viral components such as split virus, or purified surface antigens haemagglutinin (HA) and/or neuraminidase (NA)) for immunization via the intranasal route that are supported by animal data include either the use of an adjuvant or immune stimulator in combination with the inactivated influenza antigen, or require multiple vaccination. An adjuvant is any substance that enhances the immunogenicity of antigens mixed with it. In humans successful vaccination against influenza via the intranasal route has only been reported for (a) live (cold adapted strains) influenza vaccines (Flu Mist ^{™}, Medlmmune Vaccines Inc) (Refs 1, 2, 3), (b) virosomal influenza vaccine adjuvanted with the heat labile toxin of E.coli (NasalFlu, Berna Biotech Ltd) (Ref 4) or (c) using high amounts of antigen and repeated vaccination (Refs 5, 10, 11). Although live vaccines are capable of inducing a satisfactory immune response, their specific nature of being a live virus causes additional safety concerns, and is likely to induce side effects due to the required viral replication round in the upper respiratory tract. Also the required storage conditions are limiting the commercialization of these products. A strong association between the use of the intranasal influenza vaccine with E.coli HLT as adjuvant, and facial paralysis (Bell's Palsy), led to withdrawal of the HLT adjuvanted virosomal vaccine from the market (Ref 6).
The efficacy of influenza vaccines in a given population may be estimated by assessing immunogenicity parameters relating to the amount of anti-influenza antibodies that are generated after vaccination. These immunogenicity parameters, generally referred to as CHMP criteria, are used for the annual re-licensing of inactivated influenza vaccines.(Ref 7). To date, successful immunization of humans against influenza, meeting these immunology requirements or CHMP criteria (Ref 7), with one single intranasal administration of an inactivated vaccine, and without the addition of an adjuvant, being an additional ingredient of the vaccine that is not derived from the infective agent that the vaccine is intended to prevent for and that is added to the vaccine formulation for the purpose of enhancing the immune response to the antigen, has not been described. It is therefore recognized that there is still a need in the art for an inactivated influenza vaccine composition that is capable of inducing a satisfactory systemic immune response after a single intranasal administration, does not contain an adjuvant, and meets the CHMP criteria (Ref 7) with said single administration.
Said 'CHMP criteria' are defined as follows. In the CHMP (Committee for Medicinal Products for Human Use) *Note for Guidance on Harmonisation of Requirements for Influenza Vaccines*, the following serological parameters are defined to assess the immunogenicity of inactivated influenza vaccines:
- seroprotection rate, with seroprotection defined as Hemagglutination Inhibition (HI) titer ≥ 40,
- the seroconversion rate, with seroconversion defined as a pre-vaccination HI titer < 10 and a post-vaccination HI titer ≥ 40 or a pre-vaccination HI titer ≥ 10 and at least a 4-fold increase in HI titer,
- the mean fold increase, which is the geometric mean of the intra-individual increases (i.e. post-vaccination HI titer / pre-vaccination HI titer).

The CHMP requirement for influenza vaccine immunogenicity is that for each of the three virus strains in the vaccine at least one of the following criteria is met:

| criterion | adults | elderly |
|---|---|---|
| seroprotection rate: | > 70% | > 60% |
| seroconversion rate: | > 40% | > 30% |
| mean fold increase: | > 2.5 | > 2.0 |

The invention also applies to children, for whom it was shown that they respond immunologically in a comparable manner to adults (Ref 8). The invention also applies to elderly individuals. Elderly are over sixty years old.

It is herein disclosed :
1. Composition comprising influenza virosomes comprising reconstituted envelopes of said virus, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, wherein no separate adjuvant and/or immune stimulator is added to the composition, and wherein the composition is designed as an intranasal or inhalational administration formulation, which composition is characterized in that a single intranasal or inhalational administration of said formulation to a human being is capable of inducing a systemic and/or local immune response against the influenza antigens haemagglutinin and/or neuraminidase or derivatives thereof.
2. Composition according to item 1, wherein the dose of haemagglutinin per viral strain per Intranasal or Inhalational administration is lower than or equal to 30 µg.
3. Composition according to any one of items 1 or 2, wherein the single intranasal or inhalational administration of the formulation is also capable of inducing a cytotoxic lymphocytes response.
4. Composition according to any one of items 1 to 3, wherein the immune response is in accordance with the CHMP criteria for influenza vaccine.
5. Composition according to item 4, wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly.
6. Composition according to any one of items 2 to 5, wherein the dose of haemagglutinin per viral strain per intranasal or inhalational administration is lower than or equal to 25 µg.
7. Composition according to any one of items 2 to 5, wherein the dose of haemagglutinin per viral strain per intranasal or inhalational administration is lower than or equal to 20 µg.
8. Composition according to any one of items 2 to 5, wherein the dose of haemagglutinin per viral strain per intranasal or inhalational administration is lower than or equal to 15 µg.
9. Composition according to any one of items 2 to 5, wherein the dose of haemagglutinin per viral strain per intranasal or inhalational administration is lower than or equal to 10 µg.
10. Composition according to any one of items 2 to 5, wherein the dose of haemagglutinin per viral strain per intranasal or inhalational administration is lower than or equal to 5 µg.
11. Composition according to any one of items 1 to 10, wherein the composition is a vaccine formulation comprising a pharmaceutical carrier for intranasal or inhalational administration.
12. Use of influenza virosomes comprising reconstituted envelopes of said virus for the manufacture of a composition for intranasal or inhalational administration, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, and wherein no separate adjuvant and/or immune stimulator is added to the composition, which composition is characterized in that a single intranasal or inhalational administration of the composition to a human being is sufficient for the induction of a systemic and/or local immune response against the influenza antigens haemagglutinin and/or neuraminidase or derivatives thereof.
13. Use according to item 12, wherein the dose of haemagglutinin per viral strain per intranasal or inhalational administration is lower than or equal to 30 µg.
14. Use according to any one of items 12 or 13, wherein the single intranasal or inhalational administration of the composition also induces a cytotoxic lymphocytes response.
15. Use according to any one of items 12 to 14, wherein the immune response is in accordance with the CHMP criteria for influenza vaccine.
16. Use according to item 15, wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly.
17. Use according to any one of items 13 to 16, wherein the dose of haemagglutinin per viral strain per intranasal or inhalational administration is lower than or equal to 25 µg.
18. Use according to any one of items 13 to 16, wherein the dose of haemagglutinin per viral strain per intranasal or inhalational administration is lower than or equal to 20 µg.
19. Use according to any one of items 13 to 16, wherein the dose of haemagglutinin per viral strain per intranasal or inhalational administration is lower than or equal to 15 µg.
20. Use according to any one of items 13 to 16, wherein the dose of haemagglutinin per viral strain per intranasal or inhalational administration is lower than or equal to 10 µg.
21. Use according to any one of items 13 to 16, wherein the dose of haemagglutinin per viral strain per intranasal or inhalational administration is lower than or equal to 5 µg.
22. Use according to any one of items 12 to 21, wherein the manufactured composition is a vaccine formulation.
23. Vaccine formulation comprising a composition of influenza virosomes comprising reconstituted envelopes of said virus, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, wherein no separate adjuvant and/or immune stimulator is added to the composition, which vaccine is characterized in that the vaccine is designed for a single intranasal or inhalational administration to a human being and wherein the dose of haemagglutinin per viral strain per single intranasal or inhalational administration is lower than or equal to 30 µg.
24. Vaccine formulation according to item 23, wherein the dose of haemagglutinin per viral strain per single intranasal or inhalational administration is lower than or equal to 25 µg.
25. Vaccine formulation according to item 23, wherein the dose of haemagglutinin per viral strain per single intranasal or inhalational administration is lower than or equal to 20 µg.
26. Vaccine formulation according to item 23, wherein the dose of haemagglutinin per viral strain per single intranasal or inhalational administration is lower than or equal to 15 µg.
27. Vaccine formulation according to item 23, wherein the dose of haemagglutinin per viral strain per single intranasal or inhalational administration is lower than or equal to 10 µg.
28. Vaccine formulation according to item 23, wherein the dose of haemagglutinin per viral strain per single intranasal or inhalational administration is lower than or equal to 5 µg.
29. Vaccine formulation according to any one of items 23 to 28, wherein a single intranasal or inhalational administration of the formulation is capable of inducing in said human being an immune response comprising one or more of a systemic immune response against the influenza antigens haemagglutinin and/or neuraminidase or derivatives thereof, a local immune response against the influenza antigens haemagglutinin and/or neuraminidase or derivatives thereof, and a cytotoxic lymphocyte-mediated immune response.
30. Vaccine formulation according to caim 29, wherein the immune response is in accordance with the CHMP criteria for influenza vaccine.
31. Vaccine formulation according to item 30, wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly.
32. A device for intranasal or inhalational administration comprising the vaccine formulation according to any one of items 23 to 31 and a mechanism for aerosolization of the vaccine.
33. Device according to item 32, wherein the device contains a quantity of vaccine formulation for a single intranasal or inhalational administration.
34. Device according to any one of items 32 or 33, wherein the device is disposable.

In particular, the present invention relates to the therapeutic concepts as set forth in the claims and subclaims.

### Description of the Invention

The present invention provides a composition comprising influenza virosomes for use in inducing a systemic and/or local immune response according to claim 1 and a use of a composition comprising influenza virosomes in the manufacture of a vaccine formulation according to claim 6. Preferred embodiments for such composition or use are set forth in the dependent claims.

Surprisingly, and in contradiction with pre-clinical rodent data and literature on human clinical experience, we have found that the immune response in humans after a single intranasal vaccination with an inactivated influenza vaccine comprising reconstituted influenza envelopes was in accordance with all three CHMP criteria for influenza vaccine efficacy for the age group of 18-60 years old. One single intranasal administration is an inoculation of the vaccine formulation via one or both nostrils without the need for a repetition of the administration of the vaccine formulation in order to meet the above-mentioned CHMP criteria for immunogenicity of an inactivated influenza vaccine. One single administration of a vaccine (via the nasal, inhalation, oral, subcutaneous or intramuscular route) in general is a vaccination schedule which does not include multiple administrations of the vaccine that are separated in time by days or weeks known in the art as priming and boosting. A formulation designed as an intranasal or inhalational administration formulation comprises a mixture of one or more active components and excipients prepared in such a way as to allow Intranasal or inhalational administration.
The present invention provides a way of inducing a systemic immune response (circulating immunoglobulins or antibody-producing B cells) which is in accordance with the CHMP criteria, advantageously with a single intranasal or inhalational administration of virosomal influenza vaccine. The present invention also provides a way of inducing a local or mucosal immune response, comprising an increase in secretory immunoglobulins known as IgA at the surface of mucosal membranes, advantageously with a single intranasal or inhalational administration of virosomal influenza vaccine. Induction of specific IgG and IgA responses after intranasal administration involves the activity of lymphoid tissue in the nasal cavity (Ref 12). Such tissue is known as nasal-associated lymphoid tissue (NALT), and has been shown also to be a mucosal inductive site for cellular immune responses (Ref 13). Since it is known that virosomes have the potential to induce cellular immune responses (Ref 14, 15) the present invention also provides a way of inducing specific cytotoxic lymphocytes (CTL).
Virosomes are lipid bilayers containing viral glycoproteins. Virosomes are generally produced by extraction of membrane proteins and lipids from enveloped viruses with a detergent, followed by reconstitution of characteristic bilayers by removal of said detergent. The present invention also provides a composition of influenza virosomes comprising reconstituted influenza viral envelopes (in particular reconstituted without further addition of lipids and without the addition of an immunomodulator of immunostimulant (generally referred to as an adjuvant)) for the use of vaccination via an aerosol which is administrated to the mucosa of the nasopharynx or oropharynx via one or both nostrils to achieve systemic and local immunity against influenza. A single administration via inhalation is possible also. A single oral mucosal administration is possible also.
Reconstituted influenza virosomes may be prepared from inactivated virus, which may be solubilized by a non-dialyzable detergent that is removed by adsorption to hydrophobic beads. The preparation may comprise a purified suspension of one or more influenza antigens selected from haemagglutinin (HA), neuraminidase (NA), a derivative of haemagglutinin, and a derivative of neuraminidase. The viral membrane proteins haemagglutinin and neuraminidase may be reconstituted in a membrane composed of viral lipids, containing low levels of endotoxin and ovalbumin (see Ref 9). Derivatives of haemagglutinin and/or neuraminidase are haemagglutinin and/or neuraminidase molecules with modified amino acid sequences and/or structures. Amino acids may for instance be deleted, altered or added to the sequences. Also the glycosylation patterns may be altered. The derivatives retain the ability to induce an immune response when introduced into a host.
The influenza virus used for preparing the reconstituted virosomes can be grown for instance on embryonated hens' eggs or in cell culture either on adherent cells or on cells in suspension. The virus can be for instance a wild-type or a reassortant or a genetically modified strain. The virus type can for instance be any influenza A or B subtype, including pandemic strains.
The present invention also provides vaccines. The term vaccine is understood as being directed to an immunoactive pharmaceutical preparation. In certain embodiments vaccines may comprise harmless variants or derivatives of pathogenic microorganisms that, for example, stimulate the immune system to mount defenses against the actual pathogen. In certain embodiments the vaccine, for example, induces adaptive immunity when administered to a host. A vaccine may contain a dead or attenuated form of a pathogen or a component of the pathogen, such as an antigenic component of the pathogen. The vaccine preparation may further contain a pharmaceutical carrier, which may be designed for the particular mode by which the vaccine is intended to be administered, such as a pharmaceutical carrier designed for intranasal or inhalational administration. An influenza vaccine may comprise one or more non-denatured influenza antigens, one or more of which is capable of inducing an influenza-specific immune response.

The present invention provides a composition comprising influenza virosomes comprising reconstituted envelopes of said virus, wherein the composition is designed as an intranasal or inhalational administration formulation. The invention also provides said composition in which the virosomes comprise the influenza antigens haemagglutinin and/or neuraminidase or derivatives thereof. The invention also provides said composition wherein the viral envelopes are entirely derived from viral particles. The invention also provides said composition wherein no lipid is added from an external source to the reconstituted virosomes. The invention also provides said composition wherein no separate adjuvant and/or immune stimulator is added to the composition. The invention also provides said composition wherein a single intranasal or inhalational administration of the formulation to a subject is capable of inducing a systemic immune response. The invention also provides said composition wherein the single intranasal or inhalational administration of the formulation to a subject is also capable of inducing a local immune response. The invention also provides said composition wherein the single intranasal or inhalational administration of the formulation to a subject is also capable of inducing a cytotoxic lymphocytes response. The invention also provides said composition wherein the capability of inducing a systemic immune response and/or a local immune response and/or a cytotoxic lymphocytes response is exhibited in a human being. The invention also provides said composition wherein the immune response comprises an immune response directed against the influenza antigens haemagglutinin and/or neuraminidase or derivatives thereof. In a preferred embodiment, the invention also provides said composition wherein the immune response is in accordance with the CHMP criteria for influenza vaccine. The invention also provides said composition wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly. In a particularly preferred embodiment, the invention also provides said composition wherein the dose of haemagglutinin per viral strain per intranasal or inhalational administration is equal to or lower than 30 µg. Finally, the invention also provides said composition wherein the composition is a vaccine formulation comprising a pharmaceutical carrier for intranasal or inhalational administration.

The present invention further provides the use of influenza virosomes comprising reconstituted envelopes of said virus for the manufacture of a composition for intranasal or inhalational administration. The invention also provides said use in which the influenza virosomes comprise the influenza antigens haemagglutinin and/or neuraminidase or derivatives thereof. The invention also provides said use wherein the viral envelopes are entirely derived from influenza viral particles. The invention also provides said use wherein no lipid is added from an external source to the influenza virosomes in the composition. The invention also provides said use wherein no separate adjuvant and/or immune stimulator is added to the composition. The invention also provides said use wherein a single intranasal or inhalational administration of the composition to a subject is sufficient for the induction of a systemic immune response. The invention also provides said use wherein the single intranasal or inhalational administration of the composition also induces a local immune response. The invention also provides said use wherein the single intranasal or inhalational administration of the composition also induces a cytotoxic lymphocytes response. The invention also provides said use wherein the subject receiving the administration is a human being. The invention also provides said use wherein the composition induces an immune response comprising an immune response directed against the influenza antigens haemagglutinin and/or neuraminidase or derivatives thereof. In a preferred embodiment, the invention also provides said use wherein the composition induces an immune response which is in accordance with the CHMP criteria for influenza vaccine. The invention also provides said use wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly. In a particularly preferred embodiment, the invention also provides said use wherein the administered dose of haemagglutinin per viral strain per intranasal or inhalational administration is equal to or lower than 30 µg. Finally, the invention also provides said use wherein the manufactured composition is a vaccine formulation.

Thus, in an embodiment the present invention provides a composition of influenza virosomes comprising reconstituted envelopes of said virus, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, wherein no separate adjuvant and/or immune stimulator is added to the composition, and wherein the composition is designed as an intranasal or inhalational administration formulation, which composition is characterized in that a single intranasal or inhalational administration of said formulation to a human being is capable of inducing a systemic and/or local immune response against said influenza antigens, which systemic response is in accordance with the CHMP criteria for influenza vaccine, and wherein the dose of haemagglutinin per viral strain per intranasal or inhalational administration is lower than or equal to 30 µg.
In accordance with another embodiment the present invention provides the use of influenza virosomes comprising reconstituted envelopes of said virus for the manufacture of a composition for intranasal or inhalational administration, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, and wherein no separate adjuvant and/or immune stimulator is added to the composition, which use of said influenza virosomes for the manufacture of a composition for intranasal or inhalational administration is characterized in that a single intranasal or inhalational administration of the composition to a human being is sufficient for the induction of a systemic and/or local immune response against said influenza antigens, which response is in accordance with the CHMP criteria for influenza vaccine, and wherein the dose of haemagglutinin per viral strain per intranasal or inhalational administration is lower than or equal to 30 µg.
In accordance with another embodiment the present invention provides a vaccine formulation comprising a composition of influenza virosomes comprising reconstituted envelopes of said virus, wherein the viral envelopes are entirely derived from influenza viral particles, wherein no lipid is added from an external source to the reconstituted virosomes, wherein the virosomes comprise the influenza antigens haemagglutinin and/or neuraminidase or derivatives thereof, wherein no separate adjuvant and/or immune stimulator is added to the composition, which vaccine is characterized in that the vaccine is designed for a single intranasal or inhalational administration to a human being and wherein the dose of haemagglutinin per viral strain per single intranasal or inhalational administration is lower than or equal to 30 µg. Advantageously, said single intranasal or inhalational administration of the formulation is capable of inducing a systemic and/or local immune response in said human being. In accordance with the present invention there also is provided a device comprising a quantity of said vaccine formulation for a single intranasal or inhalational administration.
The applied dose according to the present invention of haemagglutinin per viral strain per intranasal or inhalational administration can also be lower than or equal to 25 µg, 20 µg, 15 µg, 10 µg, or 5 µg.

### Literature cited

(1) Maassab HF. Adaptation and growth characteristics of influenza virus at 25°C, Nature 213, 612-14 (1967)
(2) Maassab HF, Bryant ML. The development of live attenuated cold-adapted influenza virus vaccine for humans. Rev.Med.Virol. 1999 Oct-Dec;9(4):237-44
(3) Keitel W, Piedra PA. Live cold-adapted, reassortant in influenza vaccines (USA). In: Textbook of Influenza. Nicholson KG, Webster RG, Hay AJ (Ed), Blackwel Science Oxford, UK, 373-390 (1998)
(4) Gluck U, Gebbers JO, Gluck R, Phase 1 evaluation of intranasal virosomal influenza vaccine with and without Escherichia coli heat-labile toxin in adult volunteers. J Virol. 1999 Sep; 73(9):7780-6
(5) Samdal HH, Bakke H, Oftung F, Holst J, Haugen IL, Korsvold GE, Kristoffersen AC, Krogh G, Nord K, Rappuoli R, Berstad AKH, Haneberg B. Anon-Living Nasal Influenza Vaccine Can Induce Major Humoral and Cellular Immune Responses in Humans without the Need for Adjuvants. Human Vaccines 1:2, 85-90; March/April 2005
(6) Mutsch M, Zhou W, Rhodes P, Bopp M, Chen RT, Linder T, Spyr C, Steffen R. Use of the inactivated intranasal influenza vaccine and the risk of Bell's palsy in Switzerland. N Engl J Med. 2004 Feb 26;350(9):896-903
(7) Note for Guidance on Harmonisation of Requirements for Influenza Vaccines. EMEA/CpMP/BWP/214/96
(8) Daubeney, P., Taylor, C.J., McGaw, J., Brown, E.M., Ghosal, S., Keeton, B.R., Palache, B., Kerstens, R. Immunogenicity and tolerability of a trivalent influenza subunit vaccine (InfluvacR) in high-risk children aged 6 months to 4 years. BJCP 1997 March, 51 (2): 87-90
(9) Stegmann, T., Morselt, H.W.M., Booy, F.P., Van Breemen, J.F.L., Scherphof, G., Wilschut, J.Functional reconstitution of influenza viris envelopes. EMBO Journal 1987, 6(9): 2651-2659
(10) Treanor J, Nolan C, O'Brien D, Burt D, Lowell G, Linden J, Fries L. Intranasal administration of a proteosome-influenza vaccine is well-tolerated and induces serum and nasal secretion influenza antibodies in healthy human subjects. Vaccine 2006;24(3):254-62.
(11) Read R.C.,Naylor S.C.,Potter C.W.,Bond J.,Jabbal-Gill I.,Fisher A.,Illum L.,Jennings R. Effective nasal influenza vaccine delivery using chitosan. Vaccine 2005;23(35):4367-74
(12) Kuper CF, Koornstra PJ, Hameleers DM, Biewenga J, Spit BJ, Duijvestein AM, van Breda Vriesman PJ, Sminia T. The role of nasopharyngeal lymphoid tissue. Immunol. Today 1992 13:219-24
(13) Zuercher AW, Coffin SE, Thurnheer MC, Fundova P, Cebra JJ. Nasal-associated lymphoid tissue is a mucosal inductive site for virus-specific humoral and cellular immune responses. J.Immunol. 2002 168:1796-803
(14) Huckriede A, Bungener L, Stegmann T, Daemen T, Medema J, Palache AM, Wilschut J. The virosome concept for influenza vaccines. Vaccine 2005 23(Suppl 1):S26-38
(15) Glück R, Burri KG, Metcalfe I. Adjuvant and antigen delivery properties of virosomes. Curr. Drug Deliv. 2005 2:395-400

### EXAMPLES

### EXAMPLE 1

### LPP-VIROSOMAL VACCINE IN 8-WEEK-OLD BALB/C MICE; INTRANASAL COMPARISON OF VARIOUS HA/LPP RATIOS AT SUBOPTIMAL HA DOSE LEVELS

Groups of 10 influenza seronegative female Balb/c mice received LPP (lipopeptide) - virosomal vaccine by intranasal administration, at HA/LPP ratios of 1:1.5, 1:0.7, 1:0.4, 1:0 (i.e. no LPP) and with 2µg HA per dose. In addition, a control group of 10 female mice received 0 µg HA/dose (intranasal administration of vehicle).

Four preparations of LPP-containing virosomes were prepared. Briefly, inactivated influenza virus in a 30-40 % sucrose solution was sedimented by centrifugation. The virus was resuspended and solubilized in a buffer containing the detergent octaethylene glycol monododecyl ether (OEG). Subsequently, viral nucleocapsid was removed by ultracentrifugation. The OEG-containing supernatant was split in 4 equal volumes and different amounts of the lipopeptide P3CSK4 in OEG-containing buffer were added (P3CSK4: N-palmitoyl-S-[2,3-bis(palmitoyloxy)-(2RS)-propyl]-[R]-cysteinyl-[S]-seryl-[S]-lysyl-[S]-lysyl-[S]-lysyl-[S]-lysine). The volume was adjusted with OEG-containing buffer. OEG was removed by adsorption to a hydrophobic resin. This resulted in the formation of LPP-containing virosomes, reconstituted viral vesicles containing HA and NA in their membranes and (optionally) LPP in their membranes. After OEG removal the virosomes were filtered through a PVDF membrane with a pore size of 0.22 µm.

The starting material was 20 mg of HA from influenza A/Wyoming/3/2003 X-147 (A/Fujian/411/200 (H3N2)-like strain), containing 252 I.U. endotoxin/100 µg HA. After solubilization 4 batches were prepared as outlined in Table 1.

**Table 1 Preparation of virosomes**

| | | | |
|---|---|---|---|
| **Batch** | **Amount of HA as starting material (mg)** | **Amount of P3CSK4 added (mg)** | **HA/LPP Ratio** |
| VIR-2004-11 | 5 | 7.5 | 1:1.5 |
| VIR-2004-12 | 5 | 3.5 | 1:0.7 |
| VIR-2004-13 | 5 | 2.0 | 1:0.4 |
| VIR-2004-14 | 5 | 0 | 1:0 |

The vehicle was composed of 5 mM Hepes, 145 mM NaCl, 1 mM EDTA (pH 7.4). For group E (see Table 2) vehicle was filtered through a PVDF membrane with a pore size of 0.22 µm. The 4 batches of virosomes prepared were diluted to a concentration of 200 µg/ml for intranasal and 67 µg/ml for intramuscular immunization, and aliquoted in 1-ml vials (2 per group) as indicated in Table 2. These groups of vaccines were used as outlined in Table 4.

**Table 2 Preparation of vaccines**

| **Group no.** | **Preparation** |
|---|---|
| A | VIR-2004-11 |
| B | VIR-2004-12 |
| C | VIR-2004-13 |
| D | VIR-2004-14 |
| E | Vehicle* |

| | |
|---|---|
| *Vehicle: 5 mM Hepes, 145 mM NaCl, 1 mM EDTA (pH 7.4), filtered through a PVDF membrane with a pore size of 0.22 µm. | |

### Analysis of formulation

The formulations used for this study were analyzed for several variables as indicated in Table 3.

**Table 3 Analytical data on the virosomes used for preparation of the vaccines**

| **Analyte** | **VIR-2004-11** | **VIR-2004-12** | **VIR-2004-13** | **VIR-2004-14** |
|---|---|---|---|---|
| Protein (mg/ml) ^{a} | 1.7 | 1.6 | 1.5 | 1.4 |
| HA (µg/ml) ^{b} | 776 | 759 | 697 | 757 |
| Phospholipid (mmol/l) ^{c} | 0.658 | 0.692 | 0.658 | 0.682 |
| Endotoxin (I.U. per 100 µg HA) ^{d} | 3.1 | 1.5 | 1.9 | 1.0 |
| Ovalbumin (µg Per 100 µg HA)^{e} | 0.047 | 0.050 | 0.055 | 0.050 |
| Purity ^{f} | Mainly HA | Mainly HA | Mainly HA | Mainly HA |

| | | | | |
|---|---|---|---|---|
| ^{a} Lowry assay, principle: Proteins form a blue colour after treatment with alkaline copper sulphate and Folin-ciocalteu phenol reagent. The protein content is determined from the absorbance at 750 nm, using albumin BSA standard as reference. Lowry, OH, NJ Rosebrough, AL Farr, and RJ Randall. J. Biol. Chem. 193: 265. 1951. Oostra, GM, NS Mathewson, and GN Catravas. Anal. Biochem. 89: 31. 1978. Stoscheck, CM. Quantitation of Protein. Methods in Enzymology 182: 50-69 (1990). Hartree, EF. Anal Biochem 48: 422-427 (1972). ^{b} PhEur: monograph 2053 and section 2.7.1 ^{c} Principle: Each phospholipid contains a single phosphor atom, which can be used for the quantification of the phospholipids. The phospholipids are destructed by perchloric acid and the phosphate generated is complexed by molybdate that is reduced by ascorbic acid to yield a blue colored product. The color is determined with a spectrophotometer at 812 nm. The amount of phospholipid in a sample is quantified by including a phosphate calibrator. Ames BN. Assay of inorganic phosphate, total phosphate and phosphatases. Meth. Enzymol. 1966; 8:115-118 Böttcher CJF, van Gent CM & Pries C. A rapid and sensitive sub-micro phosphorus determination. Anal. Chim. Acta 1961; 24:203-204 ^{d} Ph.Eur. 2.6.14 ^{e} The Ovalbumine ELISA is a direct sandwich enzyme immunoassay using immobilized polyclonalanti-ovalbumin antibodies for capture and anti ovalbumine-HRP conjugate as detection system. Conjugate and samples are incubated simultaneously. Non bound components are removed by a washing step. Substrate (TMB and H₂O₂) is added to the wells. The presence of specifically bound conjugate in the wells is indicated by the development of a blue color. Sulphoric acid is added to the substrate to stop the reaction, and which results in a colour change in the product to yellow. Absorbances (OD) are read at 450 nm. For optimal results a reference filter at 620 nm is used. A standard curve is created from the response of ovalbumine standards (0.3-20.0 ng/ml) included in the assay. The concentrations of the unknown samples can be interpolated from the standard curve. ^{f} According to monograph 0869 and 2053: The purity of the monovalent pooled harvest is examined by polyacrylamide gel electrophoresis. Electrophoresis: according Ph.Eur 2.2.31. | | | | |

### Test system

### Test animals

Seven groups of ten female Balb/c mice (BALB/cAnNCrl) each were used.

At the initiation of treatment, the mice were 8 - 9 weeks old and weighed 17 - 19 g.

Animals were vaccinated intranasally on day 0, and day 14 with monovalent LPP virosomal influenza vaccine (A/Wyoming) and necropsied 21 days after the second vaccination.

Intranasal: test substances were inoculated intranasally (10 µl divided over both nostrils) under light Isoflurane/O₂/N₂O anaesthesia with the animal in dorsal position.

**Table 4 Treatment schedule**

| Group no. | Route of administration | Vaccine formulation | No. of females | Animal No. |
|---|---|---|---|---|
| A | Intranasal | 2 µg HA, HA/LPP ratio 1:1.5 | 10 | 01-10 |
| B | Intranasal | 2 µg HA, HA/LPP ratio 1:0.7 | 10 | 11-20 |
| C | Intranasal | 2 µg HA, HA/LPP ratio 1:0.4 | 10 | 21-30 |
| D | Intranasal | 2 µg HA, HA/LPP ratio 1:0 | 10 | 31-40 |
| E | Intranasal | 2 µg HA, HA/LPP ratio 0:0 | 10 | 41-50 |

Prior to the first vaccination and 14 days after the first vaccination, orbital blood samples were collected under Isoflurane/O₂/N₂O anaesthesia. On day 35, the animals were sacrificed and blood samples were collected (exsanguinations under O₂/CO₂ anesthesia via the abdominal aorta or cardiac puncture). Serum from all samples was harvested, deep frozen, and stored in polypropylene tubes at <-10°C until processing.

Influenza virus agglutinates red blood cells (RBCs), which is blocked in the presence of sufficient specific antibody to the virus. This phenomenon provides the basis for the hemagglutination inhibition (HI) assay, which is used to detect and quantify specific antiviral antibodies in serum. Sera were added to influenza virus and turkey RBCs. Several dilutions were tested (titration). The HI titer is defined as the reciprocal of the highest dilution that still inhibits hemagglutination. Geometrical Mean Titers (GMT) were calculated as follows:
1) calculate individual log (titer)s as the arithmetic mean of the two duplicates: [log(titer₁) + [log(titer₂)] / 2
2) calculate the arithmetic mean of the individual log (titer)s
3) GMT_{(group)} = 10 EXP (group mean log (titer)s)

### Statistics

HI titers were summarized by vaccination group and day, using geometric mean titers. Log-transformed day 35 HI titers of the groups were analyzed by means of linear regression, to investigate the dose-response relationship between the amount of LPP in the vaccine and the GMTs.

### Results

### HI titer analysis

GMTs are shown in Table 5.

**Table 5 Geometric mean titers**

| Group | Route of administration | HA/LPP ratio | Day 0 | Day 14 | Day 35 |
|---|---|---|---|---|---|
| A | Intranasal | 1:1.5 | 5 | 8 | 415 |
| B | Intranasal | 1:0.7 | 5 | 6 | 161 |
| C | Intranasal | 1:0.4 | 5 | 7 | 97 |
| D | Intranasal | 1:0 | 5 | 7 | 12 |
| E | Intranasal | 0:0 | 5 | 5 | 5 |

On day 0, no HA-specific antibodies could be detected in the mice (i.e. all HI titers < 10). On day 14, no HA-specific antibodies could be detected in most of the mice vaccinated by the intranasal route (i.n.). All HI titers were ≤ 10, except for one mouse in group A (HI titer: 80), one mouse in group C (HI titer: 35) and one mouse in group D (HI titer: 160).

On day 35 a dose-response in generation of HA-specific antibodies was observed, i.e. the addition of more LPP to the vaccine led to higher antibody titers.

The (log-transformed) day 35 HI titers were compared between the groups by means of linear regression. The fitted regression slope was highly significant (P < 0.0001). Thus, the observed dose-response relationship between the amount of LPP in the vaccine and the GMTs was statistically significant.

### Conclusion:

Repeated intranasal vaccination of mice with the non-adjuvanted reconstituted influenza virosomes did not induce a measurable systemic immune response. Repeated intranasal vaccination using LPP adjuvanted reconstituted influenza virosomes at the same HA dose level (2 µg HA/dose) and with a rising dose of LPP showed a systemic immune response in an LPP-dose depended fashion. In sharp contrast to the present invention (see Example 2 below), these data were previously considered supportive for the consensus opinion in the art that the use of an immunostimulant (in this case LPP) is essential for intranasal vaccination with inactivated influenza vaccine, even if the influenza antigen (HA) is presented in a reconstituted virosome.

### EXAMPLE 2

### A DOUBLE BLIND, RANDOMIZED, PARALLEL GROUP STUDY TO INVESTIGATE THE SAFETY OF THE LIPOPEPTIDE ADJUVANT AND ITS EFFECT ON THE EFFICACY OF A VIROSOMAL SUBUNIT INFLUENZA VACCINE AFTER NASAL DELIVERY IN HEALTHY YOUNG ADULTS AGED ≥ 18 AND ≤ 40 YEARS

Healthy human volunteers were intranasally vaccinated with LPP (lipopeptide) adjuvanted reconstituted influenza virosomes at a dose volume of 0.2ml (0.1ml per nostril) containing 150 mcg HA/mL per strain and 315 mcg LPP/mL. A similar group was intranasally vaccinated with reconstituted influenza virosomes without LPP at a dose volume of 0.2ml (0.1ml per nostril) containing 150 mcg HA/mL per strain. The objective of the study was to confirm in man the proof of concept as was shown in mice that to obtain a satisfactory systemic immune response after intranasal vaccination with an inactivated influenza vaccine the use of an adjuvant (e.g. LPP) is required.

### Study design:

This was a double-blind, randomized parallel group study in healthy young subjects aged ≥ 18 and < 40 years. The study was performed in one study center: Swiss Pharma Contract Ltd., Basel, Switzerland. The principle investigator was Dr. M. Seiberling. The study had two parts. In Part I the safety of the LPP adjuvanted virosomal subunit influenza vaccine was assessed in 12 subjects. Nine subjects were vaccinated with LPP-RVM (LPP-Reconstituted viral membranes; Influenza vaccine -surface antigen, inactivated, virosome-, adjuvanted with LPP) and three subjects with RVM (Influenza vaccine -surface antigen, inactivated, virosome-). In Part II of the study the efficacy and safety of LPP-RVM was assessed in one hundred subjects (50 per group).
The study was performed in healthy subjects. In addition, the subjects participating in Part II of the study were not vaccinated against influenza during three years previous to the start of the study. This increases the homogeneity of the study population in Part II by minimizing the number of subjects with pre-existing antibodies against influenza.

### Part I:

During 14 days prior to vaccination (Visit 1), after the subject had given informed consent, he or she was screened for in- and exclusion criteria and underwent a physical exam. At this visit a sample of nasal epithelial cells was harvested for cytology and baseline cilia activity was measured with the saccharine test.

At Visit 2 (Day 1) a 4 -6 mL blood sample was taken for standard hematology analysis, a 6 -10 mL blood sample was taken for standard biochemistry analysis and vital signs were assessed. After randomization the subject was vaccinated with one of the two vaccine formulations and remained at the site for the first twenty-four hours after vaccination to monitor immediate local and systemic reactions and adverse events. Vital signs were assessed four and twenty-four hours after vaccination. In addition, after twenty-four hours two blood samples were taken for standard hematology (4 - 6 mL) and biochemistry (6 - 10 mL) analysis; a sample of nasal epithelial cells was harvested for cytology and cilia activity after vaccination was measured with the saccharine test. Subjects were given a questionnaire (Questionnaire I) to take home to assess local and systemic reactions on the following day (Day 3).

The subject had to return to the study site two days after their release: Visit 3 (Day 4). At this visit local and systemic reactions were assessed and any spontaneous adverse events that occurred between the previous and the present visit were recorded. In addition, two blood samples were taken for standard hematology (4 - 6 mL) and biochemistry (6 - 10 mL) analysis and vital signs were assessed.

The subjects returned to the study site two weeks after the first vaccination on Day 15 (Visit 4). At this visit a sample of nasal epithelial cells was harvested for cytology, cilia activity was measured with the saccharine test and adverse events that occurred between Visit 3 and Visit 4 were recorded.

### Part II:

During 14 days prior to the first blood and nasal wash sampling (Visit 1), after the subject had given informed consent he or she was screened for in- and exclusion criteria and his or her health was checked by physical examination.

At Visit 2 (Day -1; this visit can be combined with Visit 1) a 6 to 10 mL blood sample was taken for baseline Hemagglutination Inhibition (HI) titer determination and blood samples were taken for standard hematology (4 - 6 mL) and standard biochemistry (6 - 10 mL) analysis. A nasal wash sample was collected for determination of the baseline nasal IgA antibody titer.

The following day, at Visit 3 (Day 1) after assessment of vital signs, the subject was randomized to be vaccinated with a single dose of one of the two formulations of the nasal influenza vaccine. Any immediate local reactions, systemic reactions and adverse events were monitored at the site during the first hour after vaccination. Thereafter, vital signs were reassessed and the subject received a questionnaire to take home to record daily local and systemic reactions for the first seven days after vaccination.

Two weeks later (Visit 4; Day 15), a 6 - 10 mL blood sample was taken for HI titer determination, two additional blood samples were taken for standard hematology (4 - 6 mL) and biochemistry (6 - 10 mL) analysis, a nasal wash sample was taken for nasal IgA antibody titer.

### Efficacy Assessments

To assess efficacy, blood samples and nasal wash samples were collected on Day -1 (baseline) and Day 15.

### Blood samples

Six to ten mL blood was collected to determine Hemagglutination Inhibition (HI) antibody titers.¹ After blood collection and coagulation (at least 30 minutes at room temperature) sera were separated and kept frozen (-20°C) until titration. Antibody titrations were done in duplicate. The titer assigned to a sample was the geometric mean of the two determinations. Pre- and post-vaccination sera were be titrated simultaneously.
Palmer DF, Dowdle WR, Coleman MT, Schild GC. Hemagglutination Inhibition Test. Advanced laboratory techniques for influenza diagnosis. U.S. Dept. Hlth. Ed. Welfare, P.H.S. Atlanta; 1975:25-62

### Nasal wash samples

For the collection of nasal wash samples, 6 mL of pre-warmed saline (37 °C) was applied under rhinoscopic control in one nostril. The subject was requested to incline his/her head at a 60° angle so that the washing fluid could flow. The collected wash was applied to the second nostril which was washed under the same conditions. To the samples a preservative solution was added (1/100^{th} of sample volume). The preservative solution contained 10 mg/ml bovine serum albumin dissolved in 100 mM Tris-HCl buffer, pH 8. Samples were directly clarified by low-speed centrifugation (10 min at 800xg) and aliquoted (to avoid repeated thawing of the samples later on) and placed on dry ice before transfer to - 80 °C.

IgA levels in the nasal wash samples were determined by ELISA, and statistically analyzed with the Wilcoxon test. The influenza vaccine was used as coating antigen in 96-well plates. Non-specific binding sites were blocked by incubation with a blocking buffer. The nasal washes were applied in two-fold dilutions (twelve dilutions per sample) in blocking buffer for the absorption of the influenza specific antibodies to the antigens on the 96-well plate. The 96-well plates were washed before incubation with enzyme conjugated anti-human antibodies (horse radish peroxidase or alkaline phosphatase conjugated). The non-bound anti-human antibodies were removed by washing and the amount of influenza strain specific antibody was determined by measuring the optical density after addition of the substrate for the enzymatic reaction.

### Vaccine formulation

Two different formulations of influenza vaccine were used in this study. Both formulations contained the viral antigens as recommended by the WHO for the southern hemisphere for the year 2005 at a dose level of 30mcg per strain per dose of 0.2ml
- A/New Caledonia/20/99 (H1N1)-like strain
- A/Wellington/1/2004 (H3N2)-like strain
- B/Shanghai/361/2002-like strain
² WHO. Recommended composition of influenza virus vaccines for use in the 2005 influenza season. Weekly Epidem Rec. 2004;79:369-376

Briefly, inactivated influenza virus in a 30-40 % sucrose solution was sedimented by centrifugation. The virus was resuspended and solubilized in a buffer containing the detergent octaethylene glycol monododecyl ether (OEG). Subsequently, viral nucleocapsid was removed by ultracentrifugation. The OEG-containing supernatant was adjusted with the lipopeptide P3CSK4 in OEG-containing buffer or OEG-containing buffer only in the case of LPP-free reconstituted viral membranes (P3CSK4: N-palmitoyl-S-[2,3-bis(palmitoyloxy)-(2RS)-propyl]-[R]-cysteinyl-[S]-seryl-[S]-lysyl-[S]-lysyl-[S]-lysyl-[S]-lysine). OEG was removed by adsorption to a hydrophobic resin. This resulted in the formation of LPP-containing or LPP-free reconstituted viral membranes (reconstituted viral vesicles with HA and NA in their membranes and, optionally, LPP in their membranes). After OEG removal, the virosomes were filtered through a PVDF membrane with a pore size of 0.22 µm.

For each strain of virus a separate preparation was made, either with or without LPP (Table 6). The amounts of LPP added corresponded to HA/LPP ratio's of 1:0.7 (w/w).

**Table 6 Preparation of virosomes**

| **Batch** | **LPP** | **Virus strain** |
|---|---|---|
| VIR-2005-09 | Present | Influenza B/Jiangsu/10/2003 |
| VIR-2005-11 | Present | Influenza A/New Caledonia/20/1999 IVR-116 reassortant |
| VI R-2005-13 | Present | Influenza A/Wellington/1/2004 IVR-139 reassortant |
| VIR-2005-10 | Absent | Influenza B/Jiangsu/10/2003 |
| VIR-2005-12 | Absent | Influenza A/New Caledonia/20/1999 IVR-116 reassortant |
| VI R-2005-14 | Absent | Influenza A/Wellington/1/2004 IVR-139 reassortant |

**Table 7 Analysis of formulation**

| **Analyte** | **VIR-2005-09** | **VIR-2005-10** | **VIR-2005-11** | **VIR-2005-12** | **VIR-2005-13** | **VIR-2005-14** |
|---|---|---|---|---|---|---|
| Protein (mg/ml) ^{a} | 1.51 | 1.54 | 1.86 | 1.83 | 1.37 | 1.18 |
| HA (µg/ml) ^{b} | 805 | 854 | 711 | 784 | 704 | 644 |
| Phospholipid (mmol/l) ^{c} | 0.494 | 0.563 | 0.820 | 1.03 | 0.717 | 0.695 |
| Endotoxin (I.U. per 100 µg HA) ^{d} | <0.4 | <0.4 | <0.4 | <0.4 | <0.4 | <0.5 |
| Ovalbumin (µg per 100 µg HA) ^{e} | 0.068 | 0.088 | 0.037 | 0.036 | 0.132 | 0.126 |
| Purity ^{f} PUrity | Mainly HA | Mainly HA | Mainly HA | Mainly HA | Mainly HA | Mainly HA |

| | | | | | | |
|---|---|---|---|---|---|---|
| ^{a} Lowry assay, principle: Proteins form a blue colour after treatment with alkaline copper sulphate and Folin-ciocalteu phenol reagent. The protein content is determined from the absorbance at 750 nm, using albumin BSA standard as reference. Lowry, OH, NJ Rosebrough, AL Farr, and RJ Randall. J. Biol. Chem. 193: 265. 1951. Oostra, GM, NS Mathewson, and GN Catravas. Anal. Biochem. 89: 31. 1978. Stoscheck, CM. Quantitation of Protein. Methods in Enzymology 182: 50-69 (1990). Hartree, EF. Anal Biochem 48: 422-427 (1972). ^{b} PhEur: monograph 2053 and section 2.7.1 ^{c} Principle: Each phospholipid contains a single phosphor atom, which can be used for the quantification of the phospholipids. The phospholipids are destructed by perchloric acid and the phosphate generated is complexed by molybdate that is reduced by ascorbic acid to yield a blue colored product. The color is determined with a spectrophotometer at 812 nm. The amount of phospholipid in a sample is quantified by including a phosphate calibrator. Ames BN. Assay of inorganic phosphate, total phosphate and phosphatases. Meth. Enzymol. 1966; 8:115-118 Böttcher CJF, van Gent CM & Pries C. A rapid and sensitive sub-micro phosphorus determination. Anal. Chim. Acta 1961; 24:203-204 ^{d} Ph.Eur. 2.6.14 ^{e} The Ovalbumine ELISA is a direct sandwich enzyme immunoassay using immobilized polyclonal anti-ovalbumin antibodies for capture and anti-ovalbumine-HRP conjugate as detection system. Conjugate and samples are incubated simultaneously. Non bound components are removed by a washing step. Substrate (TMB and H₂O₂) is added to the wells. The presence of specifically bound conjugate in the wells is indicated by the development of a blue color. Sulphoric acid is added to the substrate to stop the reaction, and which results in a colour change in the product to yellow. Adsorbances (OD) are read at 450 nm. For optimal results a reference filter at 620 nm is used. A standard curve is created from the response of ovalbumine standards (0.3-20.0 ng/ml) included in the assay. The concentrations of the unknown samples can be interpolated from the standard curve. ^{f} According to monograph 0869 and 2053: The purity of the monovalent pooled harvest is examined by polyacrylamide gel electrophoresis. Electrophoresis: according Ph.Eur 2.2.31 | | | | | | |

### Efficacy

Per viral strain, log-transformed HI antibody titers Day 15 and nasal IgA antibody titers at Day 15 were compared between the two vaccination groups by means of Wilcoxon's rank-sum test, at the two-sided significance level 0.05.

HI antibody titers at Day 15 were also analyzed by calculating the following three parameters per viral strain and per vaccination group:
- the seroprotection rate, with seroprotection defined as a HI titer ≥ 40
- the seroconversion rate, with seroconversion defined as a pre-vaccination HI titer < 10 and a post-vaccination Hl titer ≥ 40 or a pre-vaccination HI titer ≥ 10 and a post-vaccination rise in HI titer of at least 4-fold
- the mean fold increase, i.e. the geometric mean of the fold increases in HI titer.

The efficacy data were analyzed both according to the per-protocol and the intent-to-treat principle. However, given that this was a so-called proof-of-principle study, the per-protocol analysis was considered the primary one. The intent-to-treat sample was constituted by the vaccinated subjects with some post-vaccination efficacy data. The per-protocol sample was constituted by the vaccinated subjects who completed the protocol and for whom no major protocol violations occurred. Major violations included (amongst others): violation of the in- or exclusion criteria and use of forbidden medication. Furthermore, subjects with a laboratory confirmed intercurrent influenza infection and subjects missing primary efficacy data were also excluded from the per-protocol sample. Whether or not a subject was to be excluded from the per-protocol sample was decided before the study database was unblinded.

### Results

**Table 8 CHMP evaluation humoral immune response after nasal vaccination with the Virosomal Influenza Vaccine (RVM)**

| **Seroprotection Rates** | | | | | | | |
|---|---|---|---|---|---|---|---|
| | | A (H3N2) - like | | A (H1N1) - like | | B - like | |
| | | | | | | | |
| Statistic | | LPP-RVM | RVM | LPP-RVM | RVM | LPP-RVM | RVM |
| | | (N= 48) | (N= 43) | (N= 48) | (N= 43) | (N= 48) | (N= 43) |
| Post-Vaccination (Day 15) | | | | | | | |
| | | | | | | | |
| Seroprotection | | | | | | | |
| Yes | n(%) | 48 (100%) | 42 (97.7%) | 41 (85.4%) | 38 (88.4%) | 35 (72.9%) | 35 (81.4%) |
| No | n(%) | 0 | 1 (2.3%) | 7 (14.6%) | 5 (11.6%) | 13 (27.1%) | 8 (18.6%) |
| Total | n | 48 | 43 | 48 | 43 | 48 | 43 |
| Seroconversion | | | | | | | |
| Yes | n(%) | 37-(77.1%) | 25 (58.1%) | 25 (52.1%) | 33 (76.7%) | 24 (50.0%) | 22 (51.2%) |
| No | n(%) | 11 (22.9%) | 18 (41.9%) | 23 (47.9%) | 10 (23.3%) | 24 (50.0%) | 21 (48.8%) |
| Total | n | 48 | 43 | 48 | 43 | 48 | 43 |
| Mean Fold Increase | | | | | | | |
| n | | 48 | 43 | 48 | 43 | 48 | 43 |
| Mean* | | 12.14 | 8.52 | 4.78 | 10.07 | 3.64 | 4.51 |
| | | | | | | | |
| Note: * Geometric mean. | | | | | | | |

| | | | | | | | |
|---|---|---|---|---|---|---|---|
| RVM: Virosomal Influenza Vaccine LPP-RVM: Virosomal Influenza Vaccine adjuvanted with Lipopeptide | | | | | | | |

**Table 9 IgA titers in nasal washes (GMT)**

| | ***H3N2*** | | ***H1N1*** | | ***B*** | |
|---|---|---|---|---|---|---|
| | ***LPP-RVM*** | ***RVM*** | ***LPP-RVM*** | ***RVM*** | ***LPP-RVM*** | ***RVM*** |
| | ***N=48*** | ***N=43*** | ***N=48*** | ***N=43*** | ***N=48*** | ***N=43*** |
| ***Day 1*** | **93.88** | **96.45** | **80.93** | **85.97** | **65.84** | **55.21** |
| ***Day 15*** | **104.51** | **126.96** | **89.16** | **96.94** | **87.93** | **102.10** |

### Conclusion

Unexpectedly and in contradiction with the preclinical data obtained with the same vaccine batch (example I) and as described in WO 04/110486 and clinical data (Gluck U, Gebbers JO, Gluck R, Phase 1 evaluation of intranasal virosomal influenza vaccine with and without Escherichia coli heat-labile toxin in adult volunteers. J Virol. 1999 Sep; 73(9):7780-6), a satisfactory systemic immune response in accordance with the CHMP criteria for influenza vaccines was observed in the human group vaccinated only once with the non-adjuvanted reconstituted virosomal influenza vaccine.

## Claims

1. Composition comprising influenza virosomes comprising reconstituted envelopes of influenza virus for use in inducing a systemic and/or local immune response against the influenza antigens haemagglutinin and/or neuraminidase or derivatives thereof in a human being by means of a single intranasal or inhalational administration, wherein
• the viral envelopes are entirely derived from influenza viral particles,
• no lipid is added from an external source to the reconstituted virosomes,
• the virosomes comprise the influenza antigens haemagglutinin and/or neuraminidase or derivatives thereof, and
• no separate adjuvant and/or immune stimulator is added to the composition.

2. Composition according to claim 1, for use in inducing a cytotoxic lymphocytes response also.

3. Composition according to any one of claims 1 to 2, wherein the immune response is in accordance with the CHMP criteria for influenza vaccine.

4. Composition according to claim 3, wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly.

5. Composition according to any one of claims 1 to 4, wherein the composition is for use as a vaccine formulation comprising a pharmaceutical carrier for use in intranasal or inhalational administration.

6. Use of a composition comprising influenza virosomes comprising reconstituted envelopes of influenza virus, in the manufacture of a vaccine formulation for a single intranasal or inhalational administration inducing a systemic and/or local immune response against the influenza antigens haemagglutinin and/or neuraminidase or derivatives thereof in a human being, wherein
• the viral envelopes are entirely derived from influenza viral particles,
• no lipid is added from an external source to the reconstituted virosomes,
• the virosomes comprise the influenza antigens haemagglutinin and/or neuraminidase or derivatives thereof, and
• no separate adjuvant and/or immune stimulator is added to the composition.

7. Use according to claim 6, wherein the single intranasal or inhalational administration of the composition also induces a cytotoxic lymphocytes response.

8. Use according to any one of claims 6 to 7, wherein the immune response is in accordance with the CHMP criteria for influenza vaccine.

9. Use according to claim 8, wherein the immune response provides one or more of a seroprotection rate of > 70% for adults and/or > 60% for elderly, a seroconversion rate of > 40% for adults and/or > 30% for elderly, and a mean fold increase of > 2.5 for adults and/or > 2.0 for elderly.

## Patentansprüche

1. Zusammensetzung, umfassend Influenza-Virosome mit rekonstituierten Hüllen von Influenza-Virus, zur Verwendung in der Herbeiführung einer systemischen und/oder lokalen Immunantwort gegen die Influenza-Antigene Haemagglutinin und/oder Neuraminidase oder Derivate davon in einem Menschen durch eine einzelne intranasale oder inhalatorische Verabreichung, wobei
• die Virushüllen gänzlich von Influenzaviruspartikeln abgeleitet sind,
• kein Lipid aus einer externen Quelle zu den rekonstituierten Virosomen zugegeben ist,
• die virosome die Influenza-Antigene Haemagglutinin und/oder Neuraminidase oder Derivate davon umfassen, und
• kein Adjuvans und/oder Immunstimulator gesondert zur Zusammensetzung zugegeben ist.

2. Zusammensetzung gemäß Anspruch 1, zur Verwendung auch in der Herbeiführung einer zytotoxischen Lymphozytenantwort.

3. Zusammensetzung gemäß irgendeinem der Ansprüche 1 bis 2, wobei die Immunantwort in Übereinstimmung mit den CHMP-Kriterien für Influenza-Vakzin ist.

4. Zusammensetzung gemäß Anspruch 3, wobei die Immunantwort eine oder mehrere einer Seroprotektionsrate von >70% für Erwachsene und/oder >60% für ältere Menschen, eine Serokonversionsrate von >40% für Erwachsene und/oder >30% für ältere Menschen, und eine mittlere-fache Zunahme von >2,5 für Erwachsene und/oder >2,0 für ältere Menschen vorsieht.

5. Zusammensetzung gemäß irgendeinem der Ansprüche 1 bis 4, wobei die Zusammensetzung für eine Verwendung als eine Vakzin-Formulierung, umfassend einen pharmazeutischen Träger zur Verwendung in der intranasalen oder inhalatorischen Verabreichung, ist.

6. Verwendung einer Zusammensetzung umfassend Influenza-Virosome mit rekonstituierten Hüllen von Influenza-Virus in der Herstellung einer Vakzin-Formulierung für eine einzelne intranasale oder inhalatorische Verabreichung zum Herbeiführen einer systemischen und/oder lokalen Immunantwort gegen die Influenza-Antigene Haemagglutinin und/oder Neuraminidase oder Derivate davon in einem Menschen, wobei
• die Virushüllen gänzlich von Influenzaviruspartikeln abgeleitet sind,
• kein Lipid aus einer externen Quelle zu den rekonstituierten Virosomen zugegeben ist,
• die Virosome die Influenza-Antigene Haemagglutinin und/oder Neuraminidase oder Derivate davon umfassen, und
• kein Adjuvans und/oder Immunstimulator gesondert zur Zusammensetzung zugegeben ist.

7. Verwendung gemäß Anspruch 6, wobei die einzelne intranasale oder inhalatorische Verabreichung der Zusammensetzung auch eine zytotoxische Lymphozytenantwort herbeiführt.

8. Verwendung gemäß irgendeinem der Ansprüche 6 bis 7, wobei die Immunantwort in Übereinstimmung mit den CHMP-Kriterien für Influenza-Vakzin ist.

9. Verwendung gemäß Anspruch 8, wobei die Immunantwort eine oder mehrere einer Seroprotektionsrate von >70% für Erwachsene und/oder >60% für ältere Menschen, eine Serokonversionsrate von >40% für Erwachsene und/oder >30% für ältere Menschen, und eine mittlere-fache Zunahme von >2,5 für Erwachsene und/oder >2,0 für ältere Menschen vorsieht.

## Revendications

1. Composition comprenant des virosomes de la grippe comprenant des enveloppes reconstituées du virus de la grippe à utiliser pour induire une réponse immunitaire systémique et/ou locale contre les antigènes de la grippe hémagglutinine et/ou neuraminidase ou leurs dérivés chez un être humain, par une administration intranasale ou par inhalation unique, dans laquelle
- les enveloppes virales proviennent entièrement de particules du virus de la grippe,
- aucun lipide en provenance d'une source externe n'est ajouté aux virosomes reconstitués,
- les virosomes comprennent les antigènes de la grippe hémagglutinine et/ou neuraminidase ou leurs dérivés, et
- aucun adjuvant séparé et/ou stimulateur immunitaire n'est ajouté à la composition.

2. Composition selon la revendication 1, à utiliser également dans l'induction d'une réponse en lymphocytes cytotoxiques.

3. Composition selon l'une quelconque des revendications 1 à 2, dans laquelle la réponse immunitaire est conforme aux critères du CHMP pour le vaccin antigrippal.

4. Composition selon la revendication 3, dans laquelle la réponse immunitaire fournit un ou plus des éléments suivants : un taux de séroprotection > 70% pour les adultes et/ou > 60% pour les personnes âgées, un taux de séroconversion > 40% pour les adultes et/ou > 30% pour les personnes âgées, et un accroissement moyen de > 2,5 fois pour les adultes et/ou > 2,0 pour les personnes âgées.

5. Composition selon l'une quelconque des revendications 1 à 4, la composition étant destinée à être utilisée comme formulation vaccinale comprenant un véhicule pharmaceutique à utiliser dans l'administration intranasale ou par inhalation.

6. Utilisation d'une composition comprenant des virosomes de la grippe comprenant des enveloppes reconstituées du virus de la grippe, dans la production d'une formulation vaccinale pour une administration intranasale ou par inhalation unique induisant une réponse immunitaire systémique et/ou locale contre les antigènes de la grippe hémagglutinine et/ou neuraminidase ou leurs dérivés chez un être humain, dans laquelle
- les enveloppes virales proviennent entièrement de particules du virus de la grippe,
- aucun lipide en provenance d'une source externe n'est ajouté aux virosomes reconstitués,
- les virosomes comprennent les antigènes de la grippe hémagglutinine et/ou neuraminidase ou leurs dérivés, et
- aucun adjuvant séparé et/ou stimulateur immunitaire n'est ajouté à la composition.

7. Utilisation selon la revendication 6, dans laquelle l'administration intranasale ou par inhalation unique de la composition induit également une réponse en lymphocytes cytotoxiques.

8. Composition selon l'une quelconque des revendications 6 à 7, dans laquelle la réponse immunitaire est conforme aux critères du CHMP pour le vaccin antigrippal.

9. Utilisation selon la revendication 8, dans laquelle la réponse immunitaire fournit un ou plus des éléments suivants : un taux de séroprotection > 70% pour les adultes et/ou > 60% pour les personnes âgées, un taux de séroconversion > 40% pour les adultes et/ou > 30% pour les personnes âgées, et un accroissement moyen de > 2,5 fois pour les adultes et/ou > 2,0 pour les personnes âgées.
